(19) Europäisches Patentamt
European Patent Office
Office européen des brevets

(11) Veröffentlichungsnummer: **0 173 311**
**A1**

# (12) EUROPÄISCHE PATENTANMELDUNG

(21) Anmeldenummer: **85110822.5**

(22) Anmeldetag: **19.08.85**

(51) Int. Cl.⁴: **C 07 D 239/42**, C 07 D 401/12, C 07 D 403/12, C 07 D 405/12, C 07 D 409/12, C 07 D 251/00, C 07 D 253/04, C 07 D 253/06, C 07 D 213/00

(30) Priorität: **30.08.84 DE 3431923**

(43) Veröffentlichungstag der Anmeldung: **05.03.86 Patentblatt 86/10**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Diehr, Hans-Joachim, Dr., Hoehe 35, D-5600 Wuppertal 1 (DE)**
Erfinder: **Fest, Christa, Dr., Im Johannistal 20, D-5600 Wuppertal 1 (DE)**
Erfinder: **Kirsten, Rolf, Dr., Carl-Langhans-Strasse 27, D-4019 Monheim (DE)**
Erfinder: **Kluth, Joachim, Dr., Kurt-Schumacher-Strasse 9, D-4018 Langenfeld (DE)**
Erfinder: **Müller, Klaus-Helmut, Dr., Bockhackstrasse 55, D-4000 Düsseldorf 13 (DE)**
Erfinder: **Pfister, Theodor, Dr., Lichtenberger Strasse 30, D-4019 Monheim (DE)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**
Erfinder: **Roy, Wolfgang, Dr., Walter-Kolb-Strasse 47, D-4018 Langenfeld (DE)**

## (54) Verfahren zur Herstellung von Sulfonyliso(thio)harnstoffen.

(57) Die Erfindung betrifft ein neues Verfahren zur Herstellung von Sulfonyliso(thio)harnstoffen der allgemeinen Formel (I)

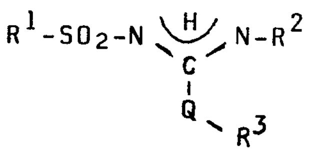

(I)

in welcher

$R^1$ für einen gegebenenfalls substituierten Rest aus der Reihe Alkyl, Aralkyl, Aryl und Heteroaryl steht,

$R^2$ für einen gegebenenfalls substituierten und/oder gegebenenfalls anellierten sechsgliedrigen aromatischen Heterocyclus, welcher wenigstens ein Stickstoffatom enthält, steht,

$R^3$ für einen gegebenenfalls substituierten Rest aus der Reihe Alkyl, Alkenyl, Alkinyl, Cycloalkyl, Cycloalkylalkyl und Aralkyl steht, und

Q für Sauerstoff oder Schwefel steht, dadurch gekennzeichnet, dass man Sulfonylguanidine der Formel (II)

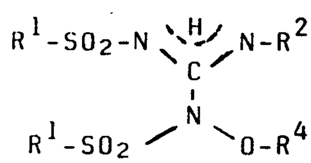

(II)

in welcher

$R^4$ für einen gegebenenfalls substituierten Kohlenwasserstoffrest steht, mit Verbindungen der Formel (III)

$H-Q-R^3$ (III)

gegebenenfalls in Gegenwart von Katalysatoren und gegebenenfalls in Gegenwart von Verdünnungsmitteln bei Temperaturen zwischen 0 °C und 150 °C umsetzt.

EP 0 173 311 A1

0 173 311

BAYER AKTIENGESELLSCHAFT
Konzernverwaltung RP
Patentabteilung

5090 Leverkusen, Bayerwerk
Bi/mö
IVa

## Verfahren zur Herstellung von Sulfonyliso(thio)harnstoffen

Die Erfindung betrifft ein neues Verfahren zur Herstellung von Sulfonyliso(thio)harnstoffen.

Es ist bekannt, daß man bestimmte Sulfonylisoharnstoffe, wie z. B. N'-(4-Methoxy-6-methyl-s-triazin-2-yl)-N''-(2-chlor-benzolsulfonyl)-O-methyl-isoharnstoff, erhält, wenn man entsprechende Sulfonylharnstoffe, wie z. B. N'-(4-Methoxy-6-methyl-s-triazin-2-yl)-N''-(2-chlor-benzolsulfonyl)-harnstoff, mit Triphenylphosphin und Tetrachlormethan und - ohne Zwischenisolierung der hierbei gebildeten N-Sulfonyl-imino-carbamidsäure-chloride - weiter mit Alkoholen, wie z. B. Methanol, oder Alkoholaten, wie z. B. Natriummethanolat, umsetzt (vergl. EP-OS 24 215). Die Sulfonylisoharnstoffe fallen hierbei jedoch stark verunreinigt an und bedürfen der Reinigung, beispielsweise durch Säulenchromatographie.

Es ist weiter bekannt, daß man bestimmte Sulfonylisothioharnstoffe, wie z. B. N'-(4-Methoxy-6-methyl-s-triazin-2-yl)-N''-(2,5-dimethoxy-benzolsulfonyl)-S-methyl-isothioharnstoff, erhält, wenn man N- Sulfonyl -imino-dithiokohlensäureester, wie z. B. N-(2,5-Dimethoxy-benzolsulfonyl)-S', S''-dimethyldithiokohlensäureester, mit Aminoverbindungen, wie z. B. 2-Amino-4-methoxy-6-methyl-s-triazin, in Gegenwart von Basen, wie z. B. Natriumhydrid, umsetzt (vergl. EP-OS 5 986).

Auch hierbei erhält man die Sulfonylisothioharnstoffe stark verunreinigt und die Reinigung ist mit größeren Ausbeuteverlusten verbunden.

Es wurde nun ein neues Verfahren zur Herstellung von Sulfonyliso(thio)harnstoffen der allgemeinen Formel (I)

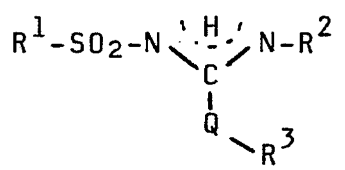

(1)

in welcher

$R^1$ für einen gegebenenfalls substituierten Rest aus der Reihe Alkyl, Aralkyl, Aryl und Heteroaryl steht,

$R^2$ für einen gegebenenfalls substituierten und/oder gegebenenfalls anellierten sechsgliedrigen aromatischen Heterocyclus, welcher wenigstens ein Stickstoffatom enthält, steht,

$R^3$ für einen gegebenenfalls substituierten Rest aus der Reihe Alkyl, Alkenyl, Alkinyl, Cycloakyl, Cycloalkylalkyl und Aralkyl steht, und

Q für Sauerstoff oder Schwefel steht,

gefunden,

welches dadurch gekennzeichnet ist, daß man Sulfonylguanidine der Formel (II)

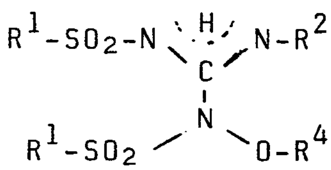

(II)

in welcher

$R^1$ und $R^2$ die oben angegebenen Bedeutungen haben und

$R^4$ für einen gegebenenfalls substituierten Kohlenwasserstoffrest steht,

mit Verbindungen der Formel (III)

$$H-Q-R^3 \qquad (III)$$

in welcher

Q und $R^3$ die oben angegebenen Bedeutungen haben,

gegebenenfalls in Gegenwart von Katalysatoren und gegebenenfalls in Gegenwart von Verdünnungsmitteln bei Temperaturen zwischen 0 °C und 150 °C umsetzt.

Es ist als überraschend anzusehen, daß die Sulfonyliso(thio)harnstoffe der Formel (I) nach dem erfindungsgemäßen Verfahren durch selektive Spaltung von Sulfonylguanidinen der Formel (II) hergestellt werden können, da neben dieser neuartigen Reaktion auch andere Spaltungsreaktionen, beispielsweise durch Angriff an den Sulfonyl-Gruppierungen zu erwarten waren.

Verwendet man als Ausgangsstoffe beispielsweise N'-(4,6-Dimethyl-pyrimidin-2-yl)-N''-methoxy-N''-N'''-bis-(2-methoxycarbonyl-benzolsulfonyl)-guanidin und Ethanthiol, so kann der Reaktionsablauf beim erfindungsgemäßen Verfahren durch folgendes Formelschema skizziert werden:

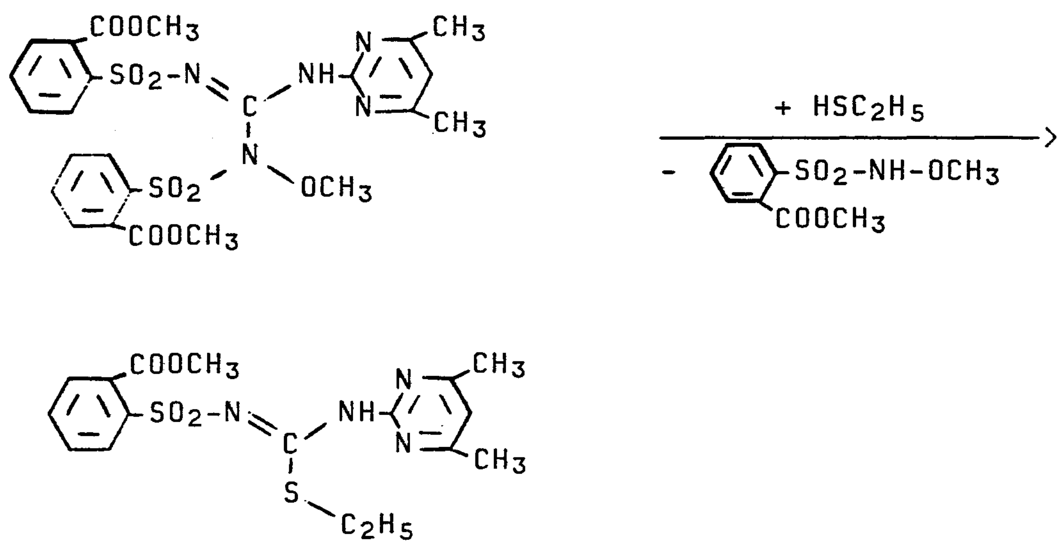


Die als Ausgangsstoffe zu verwendenden Sulfonylguanidine sind durch die Formel (II) allgemein definiert.

Vorzugsweise stehen darin

$R^1$ für den Rest 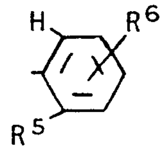 , worin

$R^5$ und $R^6$ gleich oder verschieden sind und für Wasserstoff, Halogen [wie insbesondere Fluor, Chlor und/oder Brom], Cyano, Nitro, $C_1$-$C_6$-Alkyl [welches gegebenenfalls durch Fluor, Chlor, Brom, Cyano, Carboxy, $C_1$-$C_4$-Alkoxy-carbonyl, $C_1$-$C_4$-Alkylamino-carbonyl, Di-($C_1$-$C_4$-alkyl)-amino-carbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist], für $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_1$-$C_4$-Alkoxy [welches gegebenenfalls durch Fluor, Chlor, Brom, Cyano, Carboxy, $C_1$-$C_4$-Alkoxy-carbonyl, $C_1$-$C_4$-Alkylthio,$C_1$-$C_4$-Alkylsulfinyl oder $C_1$-$C_4$-Alkylsulfonyl substituiert ist], für $C_3$-$C_6$-Alkenoxy [welches gegebenenfalls durch Fluor, Chlor, Brom, Cyano oder

$C_1$-$C_4$-Alkoxy-carbonyl substituiert ist], $C_3$-$C_6$-Alkinoxy, für den Rest $-S(O)_p-R^7$, wobei

p für die Zahlen Null, 1 oder 2 steht und

$R^7$ für $C_1$-$C_4$-Alkyl [welches gegebenenfalls durch Fluor, Chlor, Brom, Cyano oder $C_1$-$C_4$-Alkoxy-carbonyl substituiert ist], $C_3$-$C_6$-Alkenyl, $C_3$-$C_6$-Alkinyl, $C_1$-$C_4$-Alkoxy, $C_1$-$C_4$-Alkoxyamino, $C_1$-$C_4$-Alkoxy-$C_1$-$C_4$-alkyl-amino, $C_1$-$C_4$-Alkylamino oder Di-($C_1$-$C_4$-alkyl)-amino steht,

für Phenyl oder Phenoxy, für $C_1$-$C_4$-Alkyl-carbonylamino, $C_1$-$C_4$-Alkoxy-carbonylamino, $C_1$-$C_4$-Alkylamino-carbonyl-amino, Di-($C_1$-$C_4$-alkyl)-amino-carbonylamino, für den Rest $-CO-R^8$, wobei

$R^8$ für $C_1$-$C_6$-Alkyl, $C_1$-$C_6$-Alkoxy, $C_3$-$C_6$-Alkenoxy, $C_1$-$C_4$-Alkylthio, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht [welche gegebenenfalls durch Fluor und/oder Chlor substituiert sind],

für $C_1$-$C_4$-Alkylsulfonyloxy, Di-($C_1$-$C_4$-alkyl)-aminosulfonylamino oder für den

Rest $-CH=N-R^9$, wobei

$R^9$ für gegebenenfalls durch Fluor, Chlor, Cyano, Carboxy, $C_1$-$C_4$-Alkoxy, Carbonyl, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$-Alkinoxy oder Benzyloxy, für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkylsulfonylamino oder für gegebenenfalls durch Fluor, Chlor, Brom oder Methyl substituiertes Phenylsulfonylamino steht,

stehen; weiter steht vorzugsweise

$R^1$ für den Rest 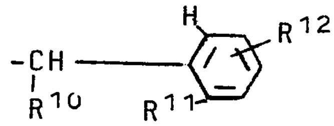 , worin

$R^{10}$ für Wasserstoff oder $C_1$-$C_3$-Alkyl steht,

$R^{11}$ und $R^{12}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], Carboxy, $C_1$-$C_4$-Alkoxy-carbonyl, $C_1$-$C_4$-Alkylsulfonyl oder Di-($C_1$-$C_4$-alkyl)-aminosulfonyl stehen; weiter steht vorzugsweise

$R^1$ für den Rest 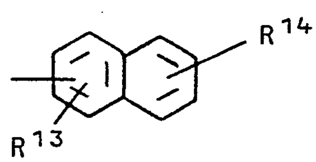 , worin

$R^{13}$ und $R^{14}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist] oder $C_1$-$C_4$-Alkoxy [welches gegebenenfalls durch Fluor und/oder Chlor substituiert ist], stehen; weiter steht vorzugsweise

$R^1$ für den Rest 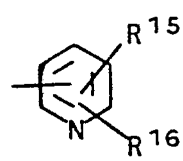 , worin

$R^{15}$ und $R^{16}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor und/oder Chlor substituiert sind], sowie für Di-($C_1$-$C_4$-alkyl)-aminosulfonyl oder $C_1$-$C_4$-Alkoxy-carbonyl stehen; weiter steht vorzugsweise

$R^1$ für den Rest 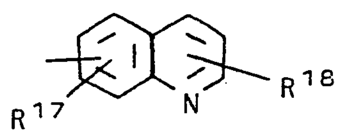 , worin

$R^{17}$ und $R^{18}$ gleich oder verschieden sind und für Wasserstoff, Fluor, Chlor, Brom, $C_1$-$C_4$-Alkyl [welches gegebenenfalls durch Fluor und/oder Brom substituiert ist], $C_1$-$C_4$-Alkoxy [welches gegebenenfalls durch Fluor und/oder Chlor substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor und/oder Chlor substituiert sind] oder für Di-($C_1$-$C_4$-alkyl)-aminosulfonyl stehen; weiter steht vorzugsweise

$R^1$ für den Rest 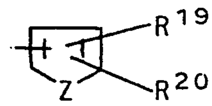 , worin

$R^{19}$ und $R^{20}$ gleich oder verschieden sind und für Wasserstoff, Fluor, Chlor, Brom, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio, $C_1$-$C_4$-Alkylsulfinyl oder $C_1$-$C_4$-Alkylsulfonyl [welches gegebenenfalls durch Fluor und/oder Chlor substituiert ist], Di-($C_1$-$C_4$-alkyl)-aminosulfonyl oder $C_1$-$C_4$-Alkoxy-carbonyl stehen, und

Z für Sauerstoff, Schwefel oder die Gruppierung N-$Z^1$ steht, wobei

$Z^1$ für Wasserstoff, $C_1$-$C_4$-Alkyl [welches gegebenenfalls durch Fluor, Chlor, Brom oder Cyano substituiert ist], $C_3$-$C_6$-Cycloalkyl, Benzyl, Phenyl [welches gegebenenfalls durch Fluor, Chlor, Brom oder Nitro substituiert ist], $C_1$-$C_4$-Alkyl-carbonyl, $C_1$-$C_4$-Alkoxy-carbonyl oder Di-($C_1$-$C_4$-alkyl)-aminocarbonyl steht;

weiter steht vorzugsweise

$R^2$ für den Rest 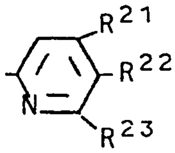 , worin

$R^{21}$ und $R^{23}$ gleich oder verschieden sind und für Wasserstoff, Fluor, Chlor, Brom, $C_1$-$C_4$-Alkyl [welches gegebenenfalls durch Fluor und/oder Chlor substituiert ist] oder $C_1$-$C_4$-Alkoxy [welches gegebenenfalls durch Fluor und/oder Chlor substiuiert ist] stehen mit der Maßgabe, daß wenigstens einer der Reste $R^{21}$ und $R^{23}$ von Wasserstoff verschieden ist, und

$R^{22}$ für Wasserstoff, Fluor, Chlor, Brom, Cyano oder $C_1$-$C_4$-Alkyl [welches gegebenenfalls durch Fluor und/oder Chlor substituiert ist] steht; weiter steht vorzugsweise

$R^2$ für den Rest 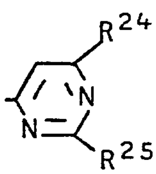 , worin

$R^{24}$ und $R^{25}$ gleich oder verschieden sind und für Wasserstoff, Fluor, Chlor, Brom, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkyl-

amino oder Di-($C_1$-$C_4$-alkyl)-amino stehen mit der Maßgabe, daß wenigstens einer der Reste $R^{24}$ und $R^{25}$ von Wasserstoff verschieden ist; weiter steht vorzugsweise

$R^2$ für den Rest 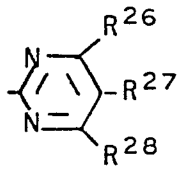 , worin

$R^{26}$ für Wasserstoff, Fluor, Chlor, Brom, Hydroxy, $C_1$-$C_4$-Alkyl [welches gegebenenfalls durch Fluor und/oder Chlor substituiert ist] oder $C_1$-$C_4$-Alkoxy [welches gegebenenfalls durch Fluor und/oder Chlor substituiert ist] steht,

$R^{27}$ für Wasserstoff, Fluor, Chlor, Brom, $C_1$-$C_4$-Alkyl [welches gegebenenfalls durch Fluor und/oder Chlor substituiert ist], Cyano, Formyl, $C_1$-$C_4$-Alkyl-carbonyl oder $C_1$-$C_4$-Alkoxy-carbonyl steht und

$R^{28}$ für Wasserstoff, Fluor, Chlor, Brom, Hydroxy, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], Amino, $C_1$-$C_4$-Alkyl-amino oder Di-($C_1$-$C_4$-alkyl)-amino steht, oder

$R^{27}$ und $R^{28}$ gemeinsam für $C_3$-$C_4$-Alkandiyl stehen; weiter steht vorzugsweise

$R^2$ für den Rest 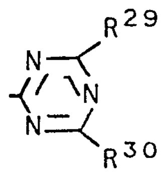 , worin

$R^{29}$ und $R^{30}$ gleich oder verschieden sind und für Fluor, Chlor, Brom, Hydroxy, $C_1$-$C_4$-Alkyl [welches gegebenenfalls durch Fluor und/oder Chlor substituiert ist], $C_3$-$C_5$-Cycloalkyl, $C_1$-$C_4$-Alkoxy [welches gegebenenfalls durch Fluor und/oder Chlor substituiert ist], $C_1$-$C_4$-Alkylthio oder für $C_1$-$C_4$-Alkyl-amino bzw. Di-($C_1$-$C_4$-Alkyl)-amino stehen; weiter steht vorzugsweise

$R^2$ für den Rest 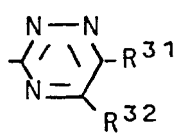 , worin

$R^{31}$ und $R^{32}$ gleich oder verschieden sind und für Wasserstoff, Methyl oder Methoxy stehen; weiter steht vorzugsweise

$R^4$ für $C_1$-$C_4$-Alkyl oder Benzyl.

Insbesondere bevorzugt sind Ausgangsstoffe der Formel (II), in welcher

(A) $R^1$ für den Rest 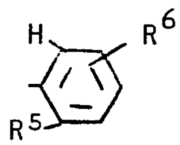 steht, worin

$R^5$ für Fluor, Chlor, Brom, Methyl, Chlormethyl, Trifluormethyl, Methoxy, Difluormethoxy, Trifluormethoxy, Methylthio, Methylsulfinyl, Methylsulfonyl, Dimethylaminosulfonyl, Phenyl, $C_1$-$C_3$-Alkoxy-carbonyl oder $C_1$-$C_3$-Alkylamino-carbonyl steht, und

$R^6$ für Wasserstoff steht; worin weiter

$R^2$ für den Rest 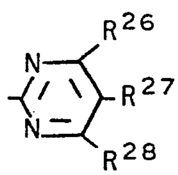 steht, worin

$R^{26}$ für Wasserstoff, Methyl, Hydroxy, Fluor, Chlor, Brom oder Methoxy steht,

$R^{27}$ für Wasserstoff, Chlor, Brom oder Methyl steht und

$R^{28}$ für $C_1$-$C_3$-Alkyl, Hydroxy, Fluor, Chlor, Brom oder $C_1$-$C_3$-Alkoxy steht; worin weiter

$R^4$ für Methyl steht,

oder in welcher

(B) $R^1$ und $R^4$ die oben unter (A) angegebene Bedeutung haben und

$R^2$ für den Rest 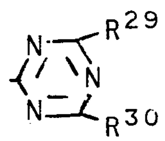 steht, worin

$R^{29}$ für Fluor, Chlor, Brom, Hydroxy, Methyl, Cyclopropyl, Methoxy, Ethoxy, Methylthio oder Ethylthio steht und

$R^{30}$ für Fluor, Chlor, Brom, Hydroxy, Methyl, Cyclopropyl, Methoxy, Ethoxy, Methylamino, Ethylamino, Dimethylamino oder Diethylamino steht.

Als Beispiele für die Ausgangsstoffe der Formel (II) seien genannt:

N'-(4,6-Dimethyl-pyrimidin-2-yl)-N''-methoxy-
-N'',N'''-bis-(2-chlor-benzolsulfonyl)-,
-N'',N'''-bis-(2-brom-benzolsulfonyl)-,
-N'',N'''-bis-(2-fluor-benzolsulfonyl)-,
-N'',N'''-bis-(2-methyl-benzolsulfonyl)-,
-N'',N'''-bis-(2-trifluormethyl-benzolsulfonyl)-,
-N'',N'''-bis-(2-methoxy-benzolsulfonyl)-,
-N'',N'''-bis-(2-phenyl-benzolsulfonyl)- und
-N'',N'''-bis-(2-methoxycarbonyl-benzolsulfonyl)-
-guanidin.

Die als Ausgangsstoffe zu verwendenden Sulfonyl-guanidine der Formel (II) sind Gegenstand einer vorgängigen, jedoch nicht zum vorveröffentlichten Stand der Technik gehörenden Patentanmeldung der Anmelderin (vgl. DE-OS 3 334 455)

Man erhält die Verbindungen der Formel (II), wenn man Guanidin-Derivate der Formel (IV),

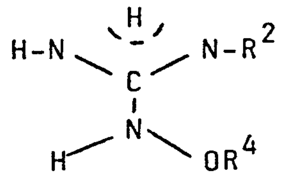

(IV)

in welcher

$R^2$ und $R^4$ die oben angegebenen Bedeutungen haben,

mit Sulfonsäurechloriden der Formel (V)

$R^1-SO_2-Cl$ (V)

in welcher

$R^1$ die oben angegebene Bedeutung hat,

in Gegenwart von Säureakzeptoren, wie z. B. Pyridin oder Diazabicyclooctan (DABCO), und gegebenenfalls in Gegenwart von Verdünnungsmitteln, wie z. B. Methylenchlorid oder Chloroform, bei Temperaturen zwischen -20 °C und +50 °C, vorzugsweise bei 0 bis 30 °C umsetzt. Die Aufarbeitung kann auf übliche Weise durchgeführt werden, beispielsweise durch Versetzen mit Wasser gegebenenfalls Ansäuern z. B. mit Salzsäure, Extrahieren mit einem mit Wasser praktisch nicht mischbaren Lösungsmittel, wie z. B. Methylenchlorid oder Chloroform, Waschen der organischen Phase mit Wasser, Trocknen, Filtrieren und Einengen. Die dabei im Rückstand verbleibenden Produkte der Formel (II) können im allgemeinen mit organischen Lösungsmitteln zur Kristallisation gebracht und gegebenenfalls durch Umkristallisieren gereinigt werden.

Die als Zwischenprodukte benötigten Guanidin-Derivate der Formel (IV) sind ebenfalls Gegenstand der oben genannten vorgängigen Patentanmeldung der Anmelderin (vergl. DE-OS 3 334 455).

Man erhält diese Guanidin-Derivate, wenn man Cyanaminover-

bindungen der Formel (VI)

$$NC-NH-R^2 \qquad (VI)$$

in welcher

$R^2$ die oben angegebene Bedeutung hat,

mit Hydroxylamin-Derivaten der Formel (VII)

$$H_2N-OR^4 \qquad (VII)$$

in welcher

$R^4$ die oben angegebene Bedeutung hat,

bzw. deren Hydrochloriden

gegebenenfalls in Gegenwart von Verdünnungsmitteln, wie z. B. Ethanol oder Butanol, bei Temperaturen zwischen 20 °C und 150 °C, vorzugsweise zwischen 50 °C und 120 °C, umsetzt und gegebenenfalls anschließend mit Säureakzeptoren, wie z. B. (wässr.) Ammoniak, Natriumhydroxid oder Kaliumcarbonat, behandelt. Die Guanidin-Derivate (IV) fallen hierbei im allgemeinen kristallin an.

Die Cyanaminoverbindungen der Formel (VI) sind zum Teil bekannt (vergl. J. Chem. Soc. 1953, 1725 - 1730). Man erhält diese Verbindungen im wesentlichen nach folgenden zwei Synthesewegen:

(1) allgemein durch Umsetzung von Alkalimetall- oder Erdalkalimetall-Salzen von Cyanamid - wie z. B. Natriumcyanamid oder Calciumcyanamid - mit Halogenverbindungen

der Formel (VIII)

$$Hal^1-R^2 \qquad (VIII)$$

in welcher

$R^2$ die oben angegebene Bedeutung hat und

$Hal^1$ für Fluor, Chlor, Brom oder Jod, insbesondere für Chlor, steht,

gegebenenfalls in Gegenwart von inerten Verdünnungsmitteln, wie z. B. Aceton, Acetonitril oder Dimethylformamid, bei Temperaturen zwischen 0 °C und 150 °C, vorzugsweise zwischen 10 °C und 100 °C; nach Abdestillieren der flüchtigen Komponente und Auflösen des Rückstandes in Wasser können die Cyanaminoverbindungen der Formel (VI) durch Ansäuern, z. B. mit Salzsäure, ausgefällt und durch Absaugen isoliert werden; oder

(2) für den Fall, daß $R^2$ für einen substituierten Pyrimidinylrest steht, durch Umsetzung von Cyanoguanidin ('Dicyandiamid') mit ß-Dicarbonylverbindungen, wie z. B. Acetylaceton (vergl. J. Chem. Soc. 1953, 1725 - 1730), Acetessigsäureester (vergl. J. Prakt. Chem. 77, (1908), 542 und J. Chem. Soc. 1948, 586) oder Malonsäureestern (vergl. DE-PS 158 591). Die aus Acetessigsäureestern bzw. Malonsäureestern erhaltenen 2-Cyanamino-4-hydroxy-6-methyl- bzw. -4,6-di-hydroxy-pyrimidine können auf bekannte Weise durch Umsetzung mit Alkylierungsmitteln, wie z. B. Dimethyl- oder Diethylsulfat, gegebenenfalls in Gegenwart von Verdünnungsmitteln, wie z. B. Wasser, Methanol, Ethanol, n- und iso-Propanol, Aceton, Dioxan oder Dimethylformamid, und in Gegenwart von Säurebindemitteln, wie z. B.

Natrium- oder Kalium-hydroxid, Natrium- oder Kaliumcarbonat, in entsprechende 2-Cyanamino-4-alkoxy-6-methyl- bzw. -4,6-dialkoxy-pyrimidine umgewandelt werden. Zur Vermeidung einer N-Alkylierung wird gegebenenfalls mit einem Acylierungsmittel, wie z. B. Acetanhydrid oder Acetylchlorid acyliert und nach der Alkylierung wird mit wässrigen Säuren oder Basen wieder entacyliert.

Die Halogenverbindungen der Formel (VIII) sind bekannt (vergl. J. Chem. Soc. (C) 1966, 2031; Chem. Pharm. Bull. 11 (1963), 1382 - 1388; Arch. Pharm. 295 (1962), 649 - 657).

Die Hydroxylamin-Derivate der Formel (VII) sind bekannt oder können nach an sich bekannten Verfahren hergestellt werden (vergl. Chem. Pharm. Bull. 15 (1967), 345 - 349; Bull. Soc. Chim. France 1958 664; Synthesis 1976, 682).

Die Sulfonsäurechloride der Formel (V) sind zum Teil bekannt (vergl. Chemistry Lett. 1978, 951; EP-PA 23 422, 35 893, 42 731, 44 808, 44 809, 51 466, 64 804 und 70 041; US-PS 2 929 820, 4 282 242 und 4 372 778; J. Org. Chem. 33 (1968), 2104).
Man erhält diese Verbindungen im wesentlichen nach folgenden zwei Synthesewegen:

(1) durch Umsetzung der entsprechenden Sulfonsäuren $R^1SO_3H$ bzw. von deren Alkalimetall- oder Erdalkalimetall-Salzen mit Halogenierungsmitteln, wie z. B. Phosphor-(V)chlorid (Phosphorpentachlorid), Phosphorylchlorid (Phosphoroxychlorid), Thionylchlorid, Phosgen oder Benzotrichlorid, gegebenenfalls in Gegenwart von Katalysatoren, wie z. B. Pyridin oder Dimethylformamid,

und gegebenenfalls unter Verwendung von inerten Verdünnungsmitteln, wie z. B. Methylenchlorid, Chloroform, Acetonitril, Chlorbenzol und/oder Sulfolan bei Temperaturen zwischen -20 °C und +150 °C, vorzugsweise zwischen 0 °C und +100 °C; nach Verdünnen mit Wasser können die Sulfonsäurechloride - soweit sie kristallin anfallen - durch Absaugen isoliert werden oder durch Extrahieren mit einem mit Wasser nicht mischbaren Lösungsmittel, wie z. B. Methylenchlorid, Diethylether oder Hexan, Waschen und Trocknen der Extrakte, Einengen und Umkristallisieren bzw. Destillieren gereinigt werden; oder auch

(2) auf an sich bekannte Weise (vergl. J. Org. Chem. 25 (1960), 1824; DE-OS 2 308 262 und EP-PA 59 241) durch Umsetzung entsprechender Aminoverbindungen $R^1-NH_2$ mit Natriumnitrit und Salzsäure, gegebenenfalls in Gegenwart von Essigsäure, bei Temperaturen zwischen -10 °C und +20 °C, vorzugsweise zwischen -5 °C und +10 °C, und anschließend (in situ) mit Schwefeldioxid oder einem Salz der schwefeligen Säure, wie z. B. Natriumsulfit oder Natriumhydrogensulfit in Gegenwart einer Kupferverbindung, wie z. B. Kupferchlorid oder Kupfersulfat, als Katalysator bei Temperaturen zwischen 0 °C und 80 °C, vorzugsweise zwischen 10 °C und 60 °C.

Die Aufarbeitung kann auf übliche Weise erfolgen: Bei Verdünnen mit Wasser fallen die Sulfonsäurechloride im allgemeinen kristallin an und können durch Absaugen isoliert werden. Sie können aber auch aus der wässrigen Dispersion mit einem mit Wasser praktisch nicht mischbaren Lösungsmittel, wie z. B. Methylenchlorid oder Diethylether, extrahiert, getrocknet und durch Vakuumdestillation gereinigt werden.

Die beim erfindungsgemäßen Verfahren weiter als Ausgangsstoffe zu verwendenden Verbindungen sind durch die Formel (III) allgemein definiert.

Vorzugsweise stehen darin

Q für Sauerstoff oder Schwefel und

$R^3$ für $C_1$-$C_{12}$-Alkyl [welches gegebenenfalls durch Fluor, Chlor, Brom, Cyano, Nitro, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist], für $C_3$-$C_6$-Alkenyl, $C_3$-$C_6$-Alkinyl, $C_3$-$C_6$-Cycloalkyl, $C_3$-$C_6$-Cycloalkyl-$C_1$-$C_2$-alkyl oder Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist].

Insbesondere bevorzugt sind Ausgangsstoffe der Formel (III), in welcher

(A) Q für Sauerstoff steht und

$R^3$ für $C_1$-$C_6$-Alkyl [welches gegebenenfalls durch Fluor, Chlor, Cyano, $C_1$-$C_2$-Alkoxy oder $C_1$-$C_2$-Alkoxy-carbonyl substituiert ist], $C_3$-$C_4$-Alkenyl, $C_3$-$C_4$-Alkinyl oder Benzyl [welches gegebenenfalls durch Fluor, Chlor, Nitro, Cyano, Methyl, Methoxy oder $C_1$-$C_2$-Alkoxy-carbonyl substituiert ist], steht oder

(B) Q für Schwefel steht und

$R^3$ die unter (A) angegebene Bedeutung hat.

Als Beispiele für die Ausgangsstoffe der Formel (III) seien genannt:

Methanol, Ethanol, Propanol, Isopropanol, Butanol, Isobutanol, sec.-Butanol, Pentanol, Isopentanol, sec.-Pentanol, Hexanol, 2-Fluor-ethanol, 2,2,2-Trifluorethanol, 2-Chlor-ethanol, 3-Chlor-propanol, 2-Cyanoethanol, 2-Methoxy-ethanol, 2-Ethoxy-ethanol, Hydroxyessigsäuremethylester, Hydroxyessigsäureethylester, Milchsäuremethylester, Milchsäureethylester, Allylalkohol, Crotylalkohol, Propargylalkohol, Benzylalkohol, 4-Fluor-, 4-Chlor-, 4-Nitro-, 4-Cyano-, 4-Methyl-, 4-Methoxy-, 4-Methoxycarbonyl- und 4-Ethoxycarbonyl-benzylalkohol sowie Methanthiol, Ethanthiol, Propanthiol, 1-Methyl-ethanthiol, Butanthiol, 2-Methyl-propanthiol, 1-Methyl-propanthiol, Mercaptoessigsäureethylester und Benzylmercaptan.

Die Ausgangsstoffe der Formel (III) sind bekannt.

Das erfindungsgemäße Verfahren wird gegebenenfalls in Gegenwart von Verdünnungsmitteln durchgeführt. Als solche kommen praktisch alle inerten organischen Lösungsmittel, vorzugsweise jedoch aprotische oder protische polare Solventien in Betracht. Hierzu gehören chlorierte Kohlenwasserstoffe, wie z. B. Methylenchlorid, Chloroform und Chlorbenzol, Ketone, wie z. B. Aceton, Methylethylketon, Methylisopropylketon und Methylisobutylketon, Nitrile, wie z. B. Acetonitril und Propionitril, Ether, wie z. B. Diethylether, Diisopropylether, Dimethoxyethan, Tetrahydrofuran und Dioxan, Amide, wie z. B. Dimethylformamid und Dimethylacetamid, sowie Dimethylsulfoxid und Sulfolan.

Soweit Hydroxyverbindungen der Formel (III) - Q steht für Sauerstoff - als Ausgangsstoffe eingesetzt werden, verwendet man vorzugsweise hiervon einen Ueberschuß und diese Verbindungen dienen dann gleichzeitig als Verdünnungsmittel.

Das erfindungsgemäße Verfahren wird gegebenenfalls in Gegenwart von Katalysatoren durchgeführt. Vorzugsweise werden Katalysatoren bei Verwendung von Hydroxyverbindungen der Formel (III) - Q steht für Sauerstoff - eingesetzt.

Geeignete Katalysatoren für das erfindungsgemäße Verfahren sind nucleophile Stickstoffverbindungen, welche praktisch keine oder nur sehr geringe Protonendonator-Eigenschaften aufweisen. Hierzu gehören Trialkylamine, wie z. B. Trimethylamin, Triethylamin, Tripropylamin und Tributylamin, Dialkyl-aralkylamine, wie z. B. N,N-Dimethyl-benzylamin und N,N-Diethylbenzylamin, Dialkyl-arylamine, wie z. B. N,N-Dimethylanilin und N,N-Diethyl-anilin, sowie Stickstoffheterocyclen, wie z. B. Pyrazol, 3,5-Dimethyl-pyrazol, Imidazol, 1-Methyl-imidazol, 2-Methyl-imidazol, Triazol, Pyridin, 4-Dimethylamino-pyridin und Diazabicylooctan (DABCO).
Die Reaktionstemperaturen können beim erfindungsgemäßen Verfahren innerhalb eines größeren Bereiches variiert werden. Im allgemeinen arbeitet man zwischen 0 °C und 150 °C, vorzugsweise zwischen 20 °C und 120 °C.

Das erfindungsgemäße Verfahren wird im allgemeinen bei Normaldruck oder geringfügig erhöhtem Druck zwischen 750 und 1000 mm Hg (997-1330 mbar) durchgeführt.

Zur Durchführung des erfindungsgemäßen Verfahrens setzt man je Mol Sulfonylguanidin der Formel (II) im allgemeinen zwischen 1 und 100 Mol, vorzugsweise zwischen 2 und 50 Mol der Verbindung der Formel (III) und gegebenenfalls zwischen 0,01 und 5 Mol, vorzugsweise zwischen 0,1 und 3 Mol eines Katalysators ein.

Die Reaktionskomponenten werden gewöhnlich bei Raumtemperatur zusammengegeben und das Reaktionsgemisch wird bis zum Reaktionsende gerührt. Soweit die Produkte hierbei kristallin anfallen, können sie durch Absaugen isoliert werden. Andernfalls wird eingeengt und der Rückstand durch Digerieren mit einem geeigneten Lösungsmittel zur Kristallisation gebracht.

Die nach dem erfindungsgemäßen Verfahren herzustellenden Sulfonyliso(thio)harnstoffe der Formel (I) können als Herbizide verwendet werden (vergl. EP-OS 5 986 und 24 215).

Herstellungsbeispiele

Beispiel 1

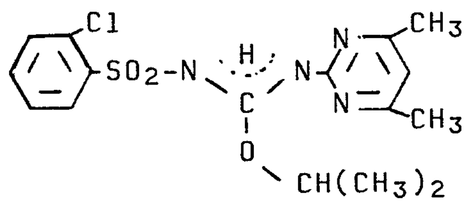

Eine Mischung aus 5,5 g (0,01 Mol) N'-(4,6-Dimethyl-pyrimidin-2-yl)-N''-methoxy-N'',N'''-bis-(2-chlorbenzolsulfonyl)-guanidin , 3,0 g (0,03 Mol) Triethylamin und 25 ml Isopropanol wird 5 Stunden bei 50 °C gerührt. Das beim Erkalten kristallin angefallene Produkt wird durch Absaugen isoliert.

Man erhält 3,0 g (78 % der Theorie) N'-(4,6-Dimethyl-pyrimidin-2-yl)-N''-(2-chlor-benzolsulfonyl)-O-(1-methylethyl)-isoharnstoff vom Schmelzpunkt 145 °C.

Beispiel 2

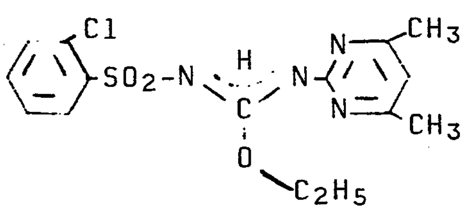

Eine Mischung aus 13,8 g (0,025 Mol) N'-(4,6-Dimethyl-pyrimidin-2-yl)-N''-methoxy-N'',N'''-bis-(2-chlor-benzolsulfonyl)-guanidin, 1,8 g (0,0265 Mol) Pyrazol und 60 ml Ethanol wird 5 Stunden unter Rückfluß zum Sieden erhitzt. Dann

wird eingeengt und der Rückstand mit Methanol verrieben. Das hierbei kristallin anfallende Produkt wird durch Absaugen isoliert.

Man erhält 4,0 g (45 % der Theorie) N'-(4,6-Dimethyl-pyrimidin-2-yl)-N''-(2-chlor-benzolsulfonyl)-O-ethyl-isoharnstoff vom Schmelzpunkt 126 °C.

Analog können die in der nachstehenden Tabelle 1 aufgeführten Verbindungen der Formel (I) hergestellt werden:

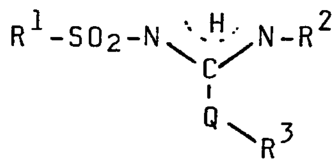

(I)

Tabelle 1

| Beisp.-Nr. | $R^1$ | $R^2$ | $R^3$ | Q | Schmelzpunkt (°C) |
|---|---|---|---|---|---|
| 3 | 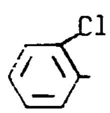 | 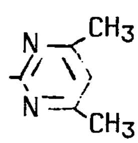 | $-CH_3$ | O | 115 |
| 4 | 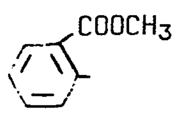 | 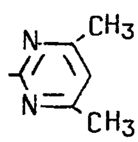 | $-CH(CH_3)_2$ | O | 83 |
| 5 | 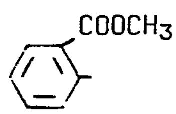 | 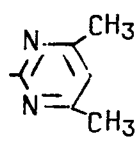 | $-CH_2CH_2Cl$ | O | 125 |

Tabelle 1 - Fortsetzung

| Beisp.-Nr. | $R^1$ | $R^2$ | $R^3$ | Q | Schmelzpunkt (°C) |
|---|---|---|---|---|---|
| 6 | 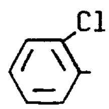 | 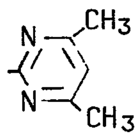 | $-CH_2CH_2Cl$ | O | 152 |
| 7 | 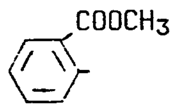 | 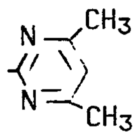 | $-CH_3$ | O | 105 |
| 8 | 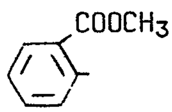 | 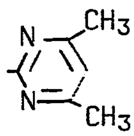 | $-CH_2CF_3$ | O | 35 |
| 9 | 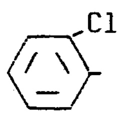 | 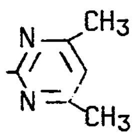 | $-CH_2COOCH_3$ | S | 202 |
| 10 | 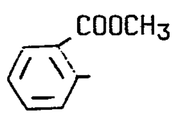 | 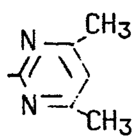 | $-C_2H_5$ | O | (amorph) |

Herstellung von Ausgangsstoffen der Formel (II)

Beispiel (II-1)

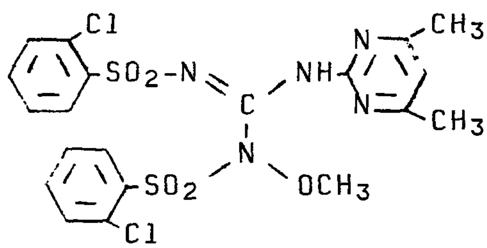

Eine Mischung aus 29,4 g (0,15 Mol) N'-(4,6-Dimethyl-pyrimidin-2-yl)-N''-methoxy-guanidin, 63,6 g (0,3 Mol) 2-Chlor-benzolsulfonsäurechlorid und 150 ml Pyridin wird 2 Tage bei 20 °C gerührt. Nach weitgehendem Abdestillieren des Pyridins im Wasserstrahlvakuum wird der Rückstand mit 200 ml Wasser versetzt und mit 200 ml Methylenchlorid extrahiert. Die organische Phase wird abgetrennt, getrocknet und eingeengt. Der Rückstand wird durch Digerieren mit Ethanol zur Kristallisation gebracht.

Man erhält 41,2 g (51 % der Theorie) N'-(4,6-Dimethyl-pyrimidin-2-yl)-N''-methoxy-N'',N'''-bis-(2-chlor-benzolsulfonyl)-guanidin vom Schmelzpunkt 164 °C bis 166 °C.

Analog können die in der nachstehenden Tabelle 2 aufgeführten Verbindungen der Formel (II) hergestellt werden:

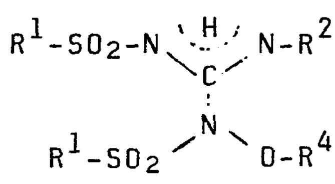

(II)

Tabelle 2

| Beisp.-Nr. | $R^1$ | $R^2$ | $R^4$ | Schmelzpunkt (°C) |
|---|---|---|---|---|
| (II-2) | 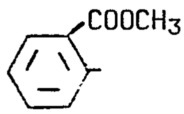 | 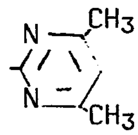 | $CH_3$ | 165 |
| (II-3) | 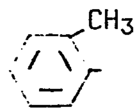 | 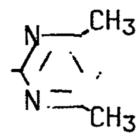 | $CH_3$ | 129 |
| (II-4) | 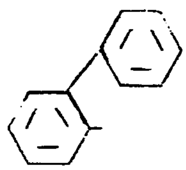 | 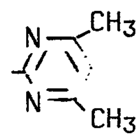 | $CH_3$ | 171 |
| (II-5) | 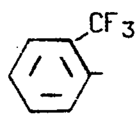 | 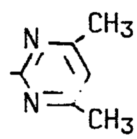 | $CH_3$ | 121 |
| (II-6) | 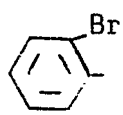 | 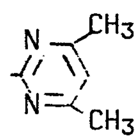 | $CH_3$ | 147 |
| (II-7) | 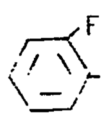 | 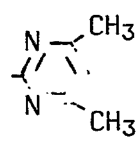 | $CH_3$ | 166 |
| (II-8) | 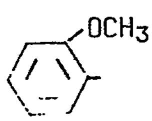 | 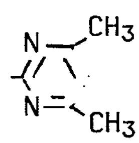 | $CH_3$ | 196 |
| (II-9) | 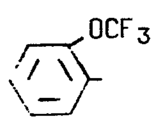 | 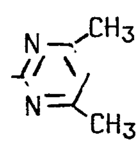 | $CH_3$ | 158 |

Tabelle 2 - Fortsetzung

| Beisp.-Nr. | $R^1$ | $R^2$ | $R^4$ | Schmelzpunkt (°C) |
|---|---|---|---|---|
| (II-10) | 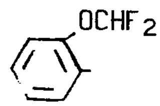 | 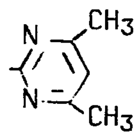 | $CH_3$ | 163 |
| (II-11) | 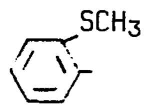 | 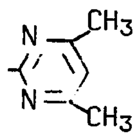 | $CH_3$ | 155 |
| (II-12) | 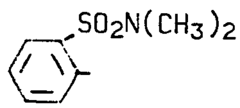 | 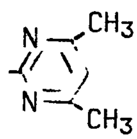 | $CH_3$ | 138 |
| (II-13) | 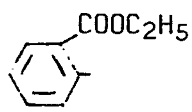 | 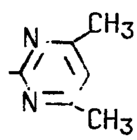 | $CH_3$ | 106 |
| (II-14) | 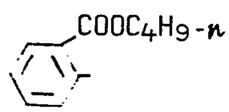 | 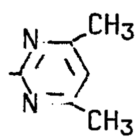 | $CH_3$ | 104 |
| (II-15) | 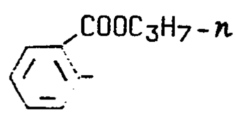 | 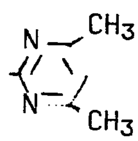 | $CH_3$ | 134 |
| (II-16) | 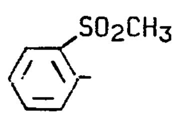 | 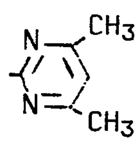 | $CH_3$ | |

Herstellung von Ausgangsstoffen der Formel (IV)

Beispiel (IV-1)

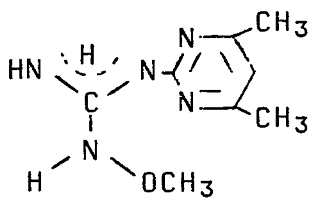

Eine Mischung aus 109 g (0,67 Mol) O-Methylhydroxylamin-Hydrochlorid, 99 g (0,67 Mol) 2-Cyanamino-4,6-dimethyl-pyrimidin und 600 ml Ethanol wird 7 Stunden unter Rückfluß zum Sieden erhitzt. Anschließend wird der Alkohol im Wasserstrahlvakuum abdestilliert, der Rückstand in heißem Wasser gelöst und diese Lösung zu 100 ml konzentriertem Ammoniak gegeben. Das auskristallisierte Produkt wird abgesaugt und aus Ethanol umkristallisiert.

Man erhält 71,8 g (55 % der Theorie) N'-(4,6-Dimethyl-pyrimidin-2-yl)-N''-methoxy-guanidin vom Schmelzpunkt 134 °C bis 136 °C.

Herstellung von Ausgangsstoffen der Formel (V)

Beispiel (V-1)

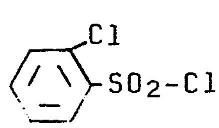

295 ml Phosphorylchlorid ('Phosphoroxychlorid') werden bei

20 °C bis 30 °C tropfenweise zu einer Mischung aus 172 g (0,8 Mol) 2-Chlor-benzolsulfonsäure-Natriumsalz, 300 ml Acetonitril und 300 ml Sulfolan gegeben. Das Reaktionsgemisch wird 4 Stunden bei 70 °C gerührt, anschließend auf 5 °C abgekühlt und mit Eiswasser verdünnt. Nach Extrahieren mit Petrolether, Waschen der Extraktionslösung mit Wasser, Trocknen, Filtrieren und Einengen wird das im Rückstand verbliebene Produkt durch Vakuumdestillation gereinigt.

Man erhält 117 g (70 % der Theorie) 2-Chlor-benzolsulfonsäurechlorid vom Siedepunkt 110 °C/ 1,1 mbar.

Beispiel (V-2)

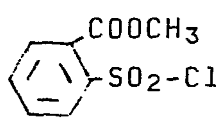

75 g (0,5 Mol) 2-Aminobenzoesäuremethylester werden in 176 ml konzentrierte Salzsäure und 100 ml Essigsäure gelöst. Hierzu wird bei 0 °C eine Lösung von 34,4 g Natriumnitrit in 70 ml Wasser getropft. Nach 15 minütigem Nachrühren wird das Reaktionsgemisch langsam zu einer auf 0 °C gekühlten gesättigten Lösung von Schwefeldioxid in 450 ml Essigsäure gegeben. Nach Entfernung des Kühlbades wird bis zum Ende der Gasentwicklung gerührt, wobei 10 g Kupfer-(II)-chlorid portionsweise eingetragen werden. Nach Verdünnen mit Eiswasser, Extrahieren mit Methylenchlorid, Waschen der Extraktionslösung mit Wasser, Trocknen, Filtrieren und Einengen wird das im Rückstand verbliebene

Produkt durch Vakuumdestillation gereinigt.

Man erhält 45 g (38 % der Theorie) 2-Methoxycarbonylbenzolsulfonsäurechlorid vom Siedepunkt 150 °C/ 1,33 mbar.

Analog können die in der nachstehenden Tabelle 3 aufgeführten Verbindungen der Formel (V) hergestellt werden:

$$R^1-SO_2-Cl \qquad (V)$$

Tabelle 3

| Beispiel-Nr. | $R^1$ | Siedepunkt/ Druck |
|---|---|---|
| (V-2) | 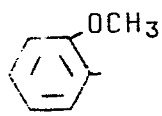 | (Oel, Zersetzung bei Destillation) |
| (V-3) | 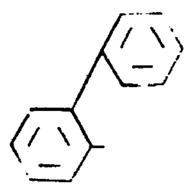 | [Schmelzpunkt: 100 °C] |
| (V-4) | 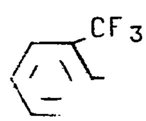 | (Oel) |
| (V-5) | 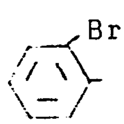 | 142 °C / 4 mbar |
| (V-6) | 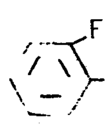 | 106 °C / 4 mbar |

Tabelle 3 - Fortsetzung

| Beispiel | $R^1$ | Siedepunkt/ Druck |
|---|---|---|
| (V-7) | 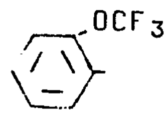 | [Schmelzpunkt: 32 °C] |
| (V-8) | 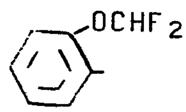 | (Oel, Zersetzung bei Destillation) |
| (V- 9) | 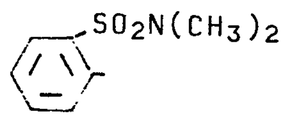 | [Schmelzpunkt: 103 °C] |
| (V-10) | 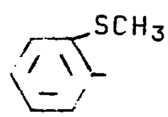 | (Oel, Zersetzung bei Destillation) |
| (V-11) | 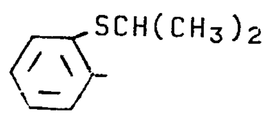 | 90 °C / 1,33 mbar |
| (V-12) | 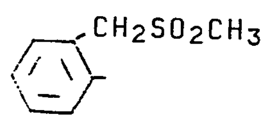 | [Schmelzpunkt: 120 °C] |
| (V-13) | 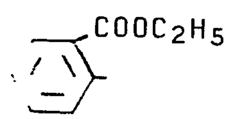 | 155 °C / 5,32 mbar |

Patentansprüche

1) Verfahren zur Herstellung von Sulfonyliso(thio)harnstoffen der allgemeinen Formel (I)

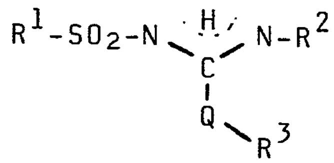

(I)

in welcher

$R^1$ für einen gegebenenfalls substituierten Rest aus der Reihe Alkyl, Aralkyl, Aryl und Heteroaryl steht,

$R^2$ für einen gegebenenfalls substituierten und/oder gegebenenfalls anellierten sechsgliedrigen aromatischen Heterocyclus, welcher wenigstens ein Stickstoffatom enthält, steht,

$R^3$ für einen gegebenenfalls substituierten Rest aus der Reihe Alkyl, Alkenyl, Alkinyl, Cycloakyl, Cycloalkylalkyl und Aralkyl steht, und

Q für Sauerstoff oder Schwefel steht,

dadurch gekennzeichnet, daß man Sulfonylguanidine der Formel (II)

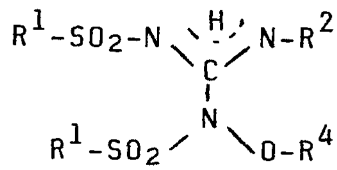

(II)

in welcher

$R^1$ und $R^2$ die oben angegebenen Bedeutungen haben und

$R^4$ für einen gegebenenfalls substituierten Kohlenwasserstoffrest steht,

mit Verbindungen der Formel (III)

$$H-Q-R^3 \qquad (III)$$

in welcher

Q und $R^3$ die oben angegebenen Bedeutungen haben,

gegebenenfalls in Gegenwart von Katalysatoren und gegebenenfalls in Gegenwart von Verdünnungsmitteln bei Temperaturen zwischen 0 °C und 150 °C umsetzt.

Europäisches Patentamt

# EUROPÄISCHER RECHERCHENBERICHT

0 173 311

EP 85110822.5

**EINSCHLÄGIGE DOKUMENTE**

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| D,A | EP - A1 - 0 024 215 (DU PONT)<br>* Anspruch 1,12 *<br>-- | 1 | C 07 D 239/42<br>C 07 D 401/12<br>C 07 D 403/12 |
| A | EP - A1 - 0 117 014 (DU PONT)<br>* Ansprüche 1,15 *<br>---- | 1 | C 07 D 405/12<br>C 07 D 409/12<br>C 07 D 251/00<br>C 07 D 253/04<br>C 07 D 253/06<br>C 07 D 213/00 |
| | | | RECHERCHIERTE SACHGEBIETE (Int. Cl 4) |
| | | | C 07 D 239/00<br>C 07 D 251/00<br>C 07 D 213/00 |

Der vorliegende Recherchenbericht wurde für alle Patentansprüche erstellt.

| Recherchenort | Abschlußdatum der Recherche | Prüfer |
|---|---|---|
| WIEN | 17-10-1985 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82